# FASCICULE DE BREVET EUROPEEN

(11) **EP 2 198 928 B1**
(45) Date de publication et mention de la délivrance du brevet: **07.11.2018**
(21) Numéro de dépôt: 09180002.9
(22) Date de dépôt: 18.12.2009
(51) Int. Cl.: A61Q 5/06, A61Q 5/08, A61Q 5/10, A61K 8/22, A61K 8/26, A61K 8/31, A61K 8/34, A61K 8/36, A61K 8/38, A61K 8/92

(54) **Composition comprenant un corps gras et un silicate, procédé de coloration la mettant en oeuvre et dispositifs**
Fettstoff und Silikat enthaltende Zusammensetzung, Verfahren zum Färben diese gebrauchend und Vorrichtungen dafür
Composition comprising a fatty substance and a silicate, dyeing process using it and devices therefore

(30) Priorité: 19.12.2008 FR 0858889
(43) Date de publication de la demande: 23.06.2010
(73) Titulaire: L'Oréal, 75008 Paris (FR)
(72) Inventeur: Simonet, Frédéric, 92110, Clichy (FR); Nicolas-Morgantini, Luc, 60810, Rully (FR); Lafuma, Aurélie, 75011, Paris (FR); Audousset, Marie-Pascale, 92600, Asnieres (FR); Rapold, Philippe, 75015, Paris (FR)
(74) Mandataire: Wattremez, Catherine

(56) Documents cités:
- EP-A1- 1 308 151
- EP-A1- 2 011 474
- WO-A1-2010/070243
- DE-A1- 3 814 356
- DE-A1- 19 909 661
- DE-A1-102006 012 575
- DE-U1- 29 923 409
- US-A- 5 817 155
- US-A1- 2005 136 098

## Description

La présente invention a pour objet une composition de coloration des fibres kératiniques humaines comprenant, outre l'agent oxydant, un colorant d'oxydation, une teneur élevée en corps gras et un silicate particulier. L'invention concerne également un procédé de coloration la mettant en oeuvre ainsi que des dispositifs à plusieurs compartiments.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs colorants d'oxydation, habituellement une ou plusieurs bases d'oxydation éventuellement associées à un ou plusieurs coupleurs.

En général, des bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

Il est également possible d'ajouter à ces compositions, des colorants directs, qui sont des molécules colorées et colorantes ayant une affinité pour les fibres. Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques. La présence de tels composés permet d'enrichir encore la coloration obtenue, en reflets ou bien d'augmenter la chromaticité de la coloration obtenue.

Les procédés de coloration d'oxydation consistent donc à employer avec ces compositions tinctoriales, une composition comprenant au moins un agent oxydant, en général le peroxyde d'hydrogène, en condition de pH alcalin dans la grande majorité des cas. Cet agent oxydant a pour rôle de révéler la coloration, par une réaction de condensation oxydative des colorants d'oxydation entre eux.

La coloration d'oxydation doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agressions extérieures telles que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (c'est-à-dire abîmées) de sa pointe à sa racine.

De nombreuses tentatives ont été faites dans le domaine de la coloration capillaire afin d'améliorer les propriétés tinctoriales, à l'aide, par exemple, d'adjuvants. Cependant, le choix de ces adjuvants est délicat dans la mesure où ils doivent améliorer les propriétés tinctoriales des compositions tinctoriales sans nuire aux autres propriétés de ces compositions. En particulier, ces adjuvants ne doivent pas nuire aux propriétés d'éclaircissement des fibres kératiniques et aux propriétés d'application de la coloration. Le document DE-A-102006012575 décrit une composition pour coloration comprenant un silicate de sodium mis en oeuvre avec des corps gras et des bases d'oxydation.

L'un des buts de la présente invention est d'obtenir des compositions pour la teinture d'oxydation des fibres kératiniques, qui ne présentent pas les inconvénients de l'art antérieur.

Plus particulièrement, l'un des buts de la présente invention est d'obtenir des compositions de coloration d'oxydation des fibres kératiniques, présentant des propriétés tinctoriales améliorées qui permettent d'atteindre l'éclaircissement souhaité et qui soient faciles à mélanger et à appliquer, notamment qui ne coulent pas et restent bien localisées au point d'application. Par propriétés tinctoriales améliorées, on entend en particulier une amélioration au niveau de la puissance/intensité, de la chromaticité et/ou de l'homogénéité de la teinture.

Ces buts et d'autres sont atteints par la présente invention qui a donc pour objet une composition de coloration de fibres kératiniques humaines, caractérisée en ce qu'elle comprend, dans un milieu cosmétiquement acceptable :
(a) au moins 25% en poids d'un ou plusieurs corps gras ;
(b) 0,1 à 15% en poids d'un ou plusieurs silicates choisis parmi les argiles de la famille des smectites, vermiculites, stévensites, chlorites;
(c) un ou plusieurs colorants d'oxydation ;
(d) un ou plusieurs agents oxydants.

Elle concerne également un procédé de coloration des fibres kératiniques humaines consistant à mettre en oeuvre la composition précitée.

L'invention a pour objet un dispositif à deux compartiments renfermant :
* dans l'un, une première composition comprenant un ou plusieurs corps gras ; un ou plusieurs colorants choisis parmi les colorants d'oxydation;
* dans l'autre, une deuxième composition comprenant un ou plusieurs agents oxydants ;
* le ou les silicates choisis parmi les argiles de la famille des smectites, vermiculites, stévensites, chlorites se trouvant dans l'une et/ou l'autre des deux compositions, et de préférence dans la première ;
* le ou les agents alcalinisants optionnels se trouvant de préférence dans la première composition ;
* les compositions des deux compartiments étant destinées à être mélangées pour donner la composition selon l'invention, juste avant l'application sur les fibres kératiniques humaines.

L'invention a de même pour objet un dispositif à deux compartiments comprenant dans l'un, une première composition comprenant un ou plusieurs corps gras, un ou plusieurs colorants d'oxydation, un ou plusieurs silicates, dans l'autre, une deuxième composition comprenant un ou plusieurs agents oxydants ; les compositions des deux compartiments étant destinées à être mélangées pour donner la composition selon l'invention, juste avant l'application sur les fibres kératiniques humaines.

L'invention concerne de plus un dispositif à trois compartiments renfermant :
* dans l'un, une première composition comprenant un ou plusieurs corps gras ;
* dans un autre, une deuxième composition renfermant un ou plusieurs colorants choisis parmi les colorants d'oxydation;
* et dans le dernier, une troisième composition comprenant un ou plusieurs agents oxydants ;
* la première et/ou la deuxième et/ou la troisième composition, et de préférence la première composition, comprenant un ou plusieurs silicates choisis parmi les argiles de la famille des smectites, vermiculites, stévensites, chlorites ;
* les compositions des trois compartiments étant destinées à être mélangées pour donner la composition selon l'invention, juste avant l'application sur les fibres kératiniques humaines.

L'invention concerne enfin un dispositif à trois compartiments comprenant dans l'un, une première composition comprenant un ou plusieurs corps gras, un ou plusieurs silicates choisis parmi les argiles de la famille des smectites, vermiculites, stévensites, chlorites, dans un autre, une deuxième composition comprenant un ou plusieurs
colorants d'oxydation, et dans le dernier, une troisième composition comprenant un ou plusieurs agents oxydants ; les compositions des trois compartiments étant destinées à être mélangées pour donner la composition selon l'invention, juste avant l'application sur les fibres kératiniques humaines.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

Comme indiqué auparavant, la composition de coloration selon l'invention comprend au moins 25% en poids d'un ou plusieurs corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0,1%). Ils présentent dans leur structure au moins une chaîne hydrocarbonée comportant au moins 6 atomes de carbone ou un enchaînement d'au moins deux groupements siloxane. En outre, les corps gras sont généralement solubles dans des solvants organiques dans les mêmes conditions de température et de pression, comme par exemple le chloroforme, l'éthanol, le benzène, l'huile de vaseline ou le décaméthylcyclopentasiloxane.

Selon l'invention, les corps gras sont choisis parmi les composés liquides ou pâteux à température ambiante et à pression atmosphérique.

Plus particulièrement, les corps gras sont choisis parmi les alcanes inférieurs en C₆-C₁₆, les huiles non siliconées d'origine animale, végétale minérale ou synthétique, les alcools gras, les acides gras, les esters d'acide gras et/ou d'alcool gras, les cires non siliconées, les silicones.

Il est rappelé qu'au sens de l'invention, les alcools, esters et acides gras présentent plus particulièrement au moins un groupement hydrocarboné, linéaire ou ramifié, saturé ou insaturé, comprenant 6 à 30 atomes de carbone, éventuellement substitué, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs en C₆-C₁₆, ces derniers sont linéaires, ramifiés, éventuellement cycliques. A titre d'exemple, on peut citer l'hexane, l'undécane, le dodécane, le tridécane, les isoparaffines comme l'isohexadécane, l'isodécane.

Comme huiles d'origine animale, végétale, minérale ou synthétique, utilisables dans la composition de l'invention, on peut citer par exemple :
- les huiles hydrocarbonées d'origine animale, telles que le perhydrosqualène.
- les huiles triglycérides d'origine végétale ou synthétique, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité.
- les hydrocarbures linéaires ou ramifiés, d'origine minérale ou synthétique, de plus de 16 atomes de carbone, tels que les huiles de paraffine, volatiles ou non, et leurs dérivés, la vaseline, l'huile de vaseline, les polydécènes, les polyisobutènes hydrogénés tels que Parléam® ; de préférence les huiles de paraffine, la vaseline, l'huile de vaseline, les polydécènes, le polyisobutène hydrogéné tel que Parléam®.
- les huiles fluorées comme le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras convenant à la mise en oeuvre de l'invention sont plus particulièrement choisis parmi les alcools saturés ou insaturés, linéaires ou ramifiés, comportant de 8 à 30 atomes de carbone. On peut citer par exemple l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

Les acides gras utilisables dans le cadre de l'invention, sont plus particulièrement choisis parmi les acides carboxyliques, saturés ou insaturés, comportant de 6 à 30 atomes de carbone, en particulier de 9 à 30 atomes de carbone. Ils sont avantageusement choisis parmi l'acide myristique, l'acide palmitique, l'acide stéarique, l'acide béhénique, l'acide oléïque, l'acide linoléïque, l'acide linolénique et l'acide isostéarique.

En ce qui concerne les esters d'acide gras et/ou d'alcools gras, avantageusement différents des triglycérides mentionnés ci-dessus ; on peut citer notamment les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total de carbone des esters étant supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et diesters de sucres d'acides gras en C₆-C₃₀, de préférence en C₁₂-C₂₂. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73% de monoester et 27% de di- et tri-ester, de 61% de monoester et 39% de di-, tri-, et tétra-ester, de 52% de monoester et 48% de di-, tri-, et tétra-ester, de 45% de monoester et 55% de di-, tri-, et tétra-ester, de 39% de monoester et 61% de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

La cire ou les cires non siliconées sont choisies notamment parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les silicones utilisables dans les compositions cosmétiques de la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.

On peut également citer les cyclocopolymères du type diméthylsiloxanes/ méthylalkylsiloxane, tel que la SILICONE VOLATILE® FZ 3109 commercialisée par la société UNION CARBIDE, de formule :

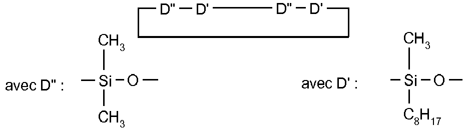

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :

R₂SiO_{2/2}, R₃SiO_{1/2}, RSiO_{3/2} et SiO_{4/2}

dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkylarylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles SILBIONE® de la série 70 641 de RHODIA;
. les huiles des séries RHODORSIL® 70 633 et 763 de RHODIA ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, les corps gras ne comprennent pas de motif oxyalkyléné en C₂-C₃ ni de motif glycérolé.

Plus particulièrement, les corps gras sont choisis parmi les composés liquides ou pâteux à température ambiante et à pression atmosphérique.

De préférence, le corps gras est un composé liquide à la température de 25°C et à la pression atmosphérique.

Plus particulièrement, les corps gras sont différents des acides gras.

Les corps gras sont de préférence choisis parmi les alcanes inférieurs en C₆-C₁₆, les huiles non siliconées d'origine végétale, minérales ou synthétique, les alcools gras, les esters d'acide gras et/ou d'alcool gras, les silicones, ou leurs mélanges, et de préférence les alcanes inférieurs en C₆-C₁₆, les huiles non siliconées d'origine végétale, minérale ou synthétique, les alcools gras, les esters d'acide gras et/ou d'alcool gras, ou leurs mélanges.

De préférence, le corps gras est choisi parmi l'huile de vaseline, les polydécènes, les esters d'acide gras et/ou d'alcool gras liquides, ou leurs mélanges.

La composition selon l'invention comprend au moins 25% de corps gras. La composition selon l'invention présente plus particulièrement une teneur en corps gras allant de 25 à 80% en poids, encore plus préférentiellement de 25 à 65%, mieux de 30 à 55% en poids par rapport au poids de la composition.

La composition selon l'invention comprend par ailleurs 0,1 à 15% d'un ou plusieurs silicates.

Les silicates de l'invention peuvent être naturels ou chimiquement modifiés (ou synthétiques).

Les silicates correspondent à de la silice éventuellement hydratée dont une partie des atomes de silicium sont remplacés par des cations métalliques comme Al³⁺, B³⁺, Fe³⁺,Ga³⁺, Be²⁺, Zn²⁺, Mg²⁺, Co³⁺, Ni³⁺, Na⁺, Li⁺, Ca²⁺, Cu²⁺.

Les silicates utilisables dans le cadre de l'invention sont choisis parmi :
- les argiles de la famille des smectites telles que les montmorillonites, les hectorites, les bentonites, les beidellites, les saponites,
- ainsi que les argiles de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques.

Le silicate peut être choisi parmi la montmorillonite, la bentonite, l'hectorite, l'attapulgite, la sépiolite, et leurs mélanges.

On peut ainsi citer les composés commercialisés par la société LAPORTE sous la dénomination LAPONITE XLG et LAPONITE XLS.

Le ou les silicates sont de préférence choisis parmi les bentonites ou les hectorites.

Le ou les silicates peuvent être modifiés avec un composé choisi parmi les amines quaternaires, les amines tertiaires, les acétates aminés, les imidazolines, les savons aminés, les sulfates gras, les alkyl aryl sulfonates, les oxides aminés, et leurs mélanges.

Comme silicates convenables, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonites telles que celles vendues sous les dénominations Claytone HT, Claytone PS par la société Southern Clay, les Quaternium-18 Hectorites telles que celles vendues sous les dénominations Bentone Gel DOA, Bentone Gel ECO5, Bentone Gel EUG, Bentone Gel IPP, Bentone Gel ISD, Bentone Gel SS71, Bentone Gel VS8, Bentone Gel VS38 par la société Rhéox et Simagel M, Simagel SI 345 par la société Biophil.

Comme indiqué auparavant, la composition selon l'invention comprend de 0,15 à 15% en poids de silicate, de préférence de 0,5 à 10 % en poids de silicate(s), par rapport au poids de la composition.

La composition selon l'invention comprend un ou plusieurs colorants choisis parmi les colorants d'oxydation qui seront détaillés ci-dessous.

Les colorants d'oxydation sont en général choisis parmi une ou plusieurs bases d'oxydation éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-((5-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE2359399 ; JP88-169571 ; JP 05-63124 ; EP0770375 ou demande de brevet WO96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE3843892, DE4133957 et demandes de brevet WO94/08969, WO94/08970, FR-A-2733749 et DE 19543988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1 -one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

La composition selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthyiamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10% en poids par rapport au poids total de la composition, et de préférence de 0,005 à 5% en poids par rapport au poids total de la composition.

La teneur en coupleur(s), s'il(s) est(sont) présent(s), représentent chacun avantageusement de 0,0001 à 10% en poids par rapport au poids total de la composition, et de préférence de 0,005 à 5% en poids par rapport au poids total de la composition.

La composition selon l'invention peut éventuellement comprendre, éventuellement en plus du ou des colorants d'oxydation et de préférence en plus du ou des colorants d'oxydation, un ou plusieurs colorants directs, synthétiques ou naturels, choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent plus particulièrement de 0,0001 à 10% en poids du poids total de la composition, et de préférence de 0,005 à 5% en poids.

La composition selon l'invention comprend également un ou plusieurs agents oxydants.

Plus particulièrement, le ou les agents oxydants sont choisis parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, ainsi que les peracides et leurs précurseurs.

De préférence, l'agent oxydant n'est pas choisi parmi les sels peroxygénés. Avantageusement, l'agent oxydant est le peroxyde d'hydrogène.

La teneur en agent(s) oxydant(s) représente plus particulièrement de 0,1 à 20% en poids, de préférence de 0,5 à 10% en poids, par rapport au poids de la composition.

L'agent alcalinisant peut être minéral ou organique ou hybride.

Le ou les agents alcalinisants minéraux sont de préférence choisis parmi l'ammoniaque, les carbonates ou bicarbonates alcalins comme les carbonates de sodium ou de potassium et les bicarbonates de sodium ou de potassium, les hydroxydes de sodium ou de potassium ou leurs mélanges.

Le ou les agents alcalinisants organiques sont de préférence choisis parmi les amines organiques dont le pKb à 25°C est inférieur à 12, et de préférence inférieur à 10, encore plus avantageusement inférieur à 6. Il est à noter qu'il s'agit du pKb correspondant à la fonction de basicité la plus élevée.

Le ou les agents alcalinisants organiques sont par exemple choisis parmi les alcanolamines, les éthylènediamines oxyéthylénées et/ou oxypropylénées, les acides aminés et les composés de formule (IX) suivante : 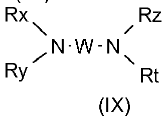 dans laquelle W est un reste alkylène en C₁-C₆ éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆, aminoalkyle en C₁-C₆.

On peut citer à titre d'exemple de telles aminés, le 1,3 diaminopropane, le 1,3 diamino 2 propanol, la spermine, la spermidine.

Par alcanolamine on entend une amine organique comprenant une fonction amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les alcanolamines telles que les mono-, di- ou tri- alcanolamines, comprenant un à trois radicaux hydroxyalkyle, identiques ou non, en C₁-C₄.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylamino-méthane.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (X) suivante : 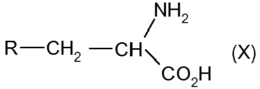 où R désigne un groupe choisi parmi :

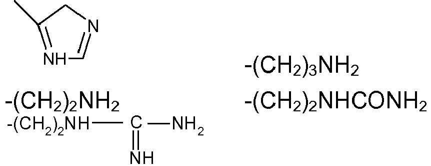

Les composés correspondants à la formule (X) sont l'histidine, la lysine, l'arginine, l'ornithine, la citrulline.

L'amine organique peut être aussi choisie parmi les amines organiques de type hétérocycliques On peut en particulier citer, outre l'histidine déjà mentionnée dans les acides aminés, la pyridine, la pipéridine, l'imidazole, le triazole, le tétrazole, le benzimidazole.

L'amine organique peut être aussi choisie parmi les dipeptides d'acides aminés. A titre de dipeptides d'acides aminés utilisables dans la présente invention, on peut notamment citer la carnosine, l'anserine et la baleine

L'amine organique est choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)-methyl]amino)ethane-1-sulfonique.

De préférence l'amine organique présente dans la composition de l'invention est une alcanolamine.

Encore plus préférentiellement l'amine organique est la monoéthanolamine.

A titre de composés hybrides on peut mentionner les sels des amines citées précédemment avec des acides comme l'acide carbonique, l'acide chlorhydrique.

On peut en particulier utiliser le carbonate de guanidine ou le chlorhydrate de monoéthanolamine.I

De manière avantageuse, la composition selon l'invention présente une teneur en agent(s) alcalinisant(s) allant de 0,01 à 30 % en poids, de préférence de 0,1 à 20 % en poids par rapport au poids de ladite composition.

Il est à noter que, de préférence, la composition selon l'invention ne comprend pas d'ammoniaque ou un de ses sels, en tant qu'agent alcalinisant. Si toutefois elle en comprenait, sa teneur ne dépasserait pas 0,03 % en poids (exprimé en NH₃), de préférence ne dépasserait pas 0,01 % en poids, par rapport au poids de la composition selon l'invention.. De préférence, si la composition comprend de l'ammoniaque ou un de ses sels, alors la quantité d'agent(s) alcalinisant(s) est supérieure à celle d'ammoniaque (exprimé en NH₃).

La composition de l'invention contient de préférence une ou plusieurs alcanolamines, et/ou un ou plusieurs acides aminés basiques.

De préférence la composition de l'invention contient de la monoéthanolamine.

La composition selon l'invention peut également un ou plusieurs tensioactifs.

De préférence, le ou les tensioactifs sont choisis parmi les tensioactifs non ioniques ou parmi les tensioactifs anioniques.

Les tensioactifs anioniques sont plus spécialement choisis parmi les sels (en particulier sels de métaux alcalins, notamment de sodium, sels d'ammonium, sels d'amines, sels d'aminoalcools ou sels de métaux alcalino-terreux comme le magnésium) des composés suivants :
- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Les tensioactifs présentant un nombre de moles d'oxyde d'éthylène et/ou de propylène compris entre 1 et 100, plus particulièrement entre 2 et 50, de préférence entre 2 et 30. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀, oxyéthylénés comprenant de 1 à 100 moles d'oxyde d'éthylène; les esters d'acides en C₈-C₃₀, saturés ou non, linéaires ou ramifiés, et de sorbitol polyoxyéthylénés comprenant de 1 à 100 moles d'oxyde d'éthylène.

A titre d'exemple de tensioactifs non ioniques mono- ou poly- glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

De préférence, le tensioactif présent dans la composition est un tensioactif non ionique.

La teneur en tensioactifs dans la composition représente plus particulièrement de 0,1 à 50% en poids, de préférence de 0,5 à 30% en poids par rapport au poids de la composition.

La composition peut également renfermer divers adjuvants utilisés classiquement dans les compositions pour la coloration des cheveux, tels que des polymères anioniques, cationiques, non ioniques, amphotères, zwitterioniques ou leurs mélanges ; des agents antioxydants ; des agents de pénétration ; des agents séquestrants ; des parfums ; des agents dispersants ; des agents filmogènes ; des céramides ; des agents conservateurs ; des agents opacifiants.

Les adjuvants ci dessus sont en général présents en quantité comprise pour chacun d'eux entre 0,01 et 20% en poids par rapport au poids de la composition.

La composition peut comprendre une ou plusieurs silices pyrogénées.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de la silice par réaction chimique en vue de diminuer le nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

Lorsqu'elle est présente, la silice pyrogénée représente de 1 à 30 % en poids par rapport au poids de la composition.

La composition peut également comprendre un ou plusieurs agents épaississants organiques.

Ces agents épaississants peuvent être choisis parmi les amides d'acides gras (diéthanol- ou monoéthanol-amide de coprah, monoéthanolamide d'acide alkyl éther carboxylique oxyéthyléné), les épaississants polymériques tels que les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs (polymères comprenant des zones hydrophiles, et des zones hydrophobes à chaîne grasse (alkyle, alcényle comprenant au moins 10 atomes de carbone) capables, dans un milieu aqueux, de s'associer réversiblement entre eux ou avec d'autres molécules.).

Selon un mode de réalisation particulier, l'épaississant organique est choisi parmi les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique, et de préférence parmi les épaississants cellulosiques avec en particulier l'hydroxyéthycellulose.

La teneur en agent(s) épaississant(s) organique(s), s'ils sont présents, varie habituellement de 0,01% à 20% en poids, par rapport au poids de la composition, de préférence de 0,1 à 5% en poids.

Le milieu cosmétiquement acceptable de la composition selon l'invention est un milieu comprenant au moins de l'eau et éventuellement un ou plusieurs solvants organiques.

A titre de solvant organique, on peut par exemple citer les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; le glycérol ; les polyols ou éthers de polyol tels que, par exemple, les éthers monométhylique, monoéthylique et monobutylique d'éthylèneglycol, le 2-butoxyéthanol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants, quand il sont présents, représentent généralement entre 1 et 40% en poids par rapport au poids total de la composition tinctoriale, et de préférence entre 5 et 30% en poids par rapport au poids total de la composition tinctoriale.

De préférence la composition de l'invention contient de l'eau.

Avantageusement la concentration en eau peut aller de 10 à 70% en poids, de préférence de 20 à 55% du poids total de la composition.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Avantageusement, la composition selon l'invention se présente sous la forme d'un gel ou d'une crème.

Le pH de la composition selon l'invention est avantageusement compris entre 3 et 12, de préférence entre 5 et 11. Préférentiellement entre 7 et 11 bornes comprises.

Il peut être ajusté à la valeur désirée au moyen d'agents acidifiants ou alcalinisants habituellement utilisés en teinture des fibres kératiniques ou bien encore à l'aide de systèmes tampons classiques.

Les agents alcalins sont par exemple ceux décrits précédemment.

Parmi les agents acidifiants, on peut citer, à titre d'exemple, des acides minéraux ou organiques comme l'acide chlorhydrique, l'acide ortho-phosphorique, des acides carboxyliques comme l'acide tartrique, l'acide citrique, l'acide lactique, ou des acides sulfoniques.

La composition de l'invention peut être obtenue par mélange d'au moins deux compositions différentes, voire trois ou éventuellement plus de trois compositions différentes.

Une ou plusieurs des compositions conduisant par mélange à la composition de l'invention peuvent être anhydres.

Par compositions anhydres, on entend plus particulièrement des compositions dont la teneur en eau est égale à 0 ou inférieure à 5% en poids, de préférence inférieure à 2% en poids, et de manière encore plus particulière inférieure à 1% en poids, par rapport au poids de ladite composition. Il est à noter que l'eau peut aussi se trouver sous forme d'eau liée, comme l'eau de cristallisation des sels ou des traces d'eau absorbée par les matières premières utilisées dans la réalisation des compositions selon l'invention.

A noter que la composition selon l'invention est préparée juste avant son application sur les fibres kératiniques humaines.

Selon une première variante, la composition selon l'invention est obtenue en mélangeant une première composition comprenant un ou plusieurs corps gras, un ou plusieurs silicates particuliers, éventuellement un ou plusieurs colorants ; avec une deuxième composition comprenant un ou plusieurs agents oxydants ; le ou les éventuels agents alcalinisants se trouvant dans la première composition et/ou dans la deuxième composition, et de préférence dans la première composition ; et la concentration finale en silicates allant de 0,1 à 15% en poids.

Selon une variante, la composition selon l'invention est obtenue en mélangeant une première composition comprenant un ou plusieurs corps gras, un ou plusieurs silicates particuliers, un ou plusieurs colorants d'oxydation, avec une deuxième composition comprenant un ou plusieurs agents oxydants ; le ou les éventuels agents alcalinisants se trouvant également dans la première composition et/ou dans la deuxième composition, et de préférence dans la première composition ; et la concentration finale en silicates allant de 0,1 à 15% en poids.

Selon une deuxième variante de l'invention, la composition selon l'invention est obtenue en mélangeant une première composition comprenant un ou plusieurs corps gras, un ou plusieurs silicates particuliers ; une deuxième composition comprenant un ou plusieurs colorants, un ou plusieurs agents alcalinisants, ou leurs mélanges ; et une troisième composition comprenant un ou plusieurs agents oxydants ; la concentration finale en silicates allant de 0,1 à 15% en poids.

La première composition peut en particulier être anhydre.

Le ou les éventuels agents alcalinisants se trouvent dans l'une ou l'autre des trois compositions précitées, et de préférence dans la première composition.

Selon une variante additionnelle de l'invention, la composition selon l'invention est obtenue en mélangeant une première composition comprenant un ou plusieurs corps gras, un ou plusieurs silicates particuliers, une deuxième composition comprenant un ou plusieurs colorants d'oxydation, et une troisième composition comprenant un ou plusieurs un agent oxydant ; le ou les éventuels agents alcalinisants se trouvant également dans l'une ou l'autre des trois compositions précitées, et de préférence dans la première composition ; et la concentration finale en silicates allant de 0,1 à 15% en poids. La première composition peut en particulier être anhydre.

Les ingrédients des compositions précitées et leurs teneurs sont déterminés en fonction des caractéristiques détaillées auparavant pour la composition finale selon l'invention.

Dans chacune des variantes précitées, la composition oxydante est de préférence une composition aqueuse. En particulier, elle comprend plus de 5% en poids d'eau, de préférence plus de 10% en poids d'eau, et de manière encore plus avantageuse plus de 20 % en poids d'eau.

Elle peut également comprendre un ou plusieurs solvants organiques choisis parmi ceux listés auparavant ; ces derniers représentant plus particulièrement, lorsqu'ils sont présents, de 1 à 40% en poids par rapport au poids de la composition oxydante, et de préférence de 5 à 30% en poids.

La composition oxydante comprend également de manière préférée, un ou plusieurs agents acidifiants. Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition oxydante, lorsqu'elle est aqueuse, est inférieur à 7.

De préférence, la composition oxydante comprend du peroxyde d'hydrogène en tant qu'agent oxydant, en solution aqueuse, dont la concentration varie, plus particulièrement, de 0,1 à 50% en poids, plus particulièrement entre 0,5 et 20% en poids, et encore plus préférentiellement entre 1 et 15 % en poids, par rapport au poids de la composition oxydante.

Le procédé de coloration selon l'invention consiste donc à appliquer la composition selon l'invention, sur les fibres kératiniques humaines, sèches ou humides.

La composition est ensuite laissée en place pendant une durée allant habituellement d'une minute à une heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les fibres kératiniques humaines sont éventuellement rincées à l'eau, subissent éventuellement un lavage avec un shampooing suivi d'un rinçage à l'eau, avant d'être séchées ou laissées sécher.

L'invention a pour objet un dispositif à deux compartiments renfermant :
* dans l'un, une première composition comprenant un ou plusieurs corps gras ; un ou plusieurs colorants choisis parmi les colorants d'oxydation;
* dans l'autre, une deuxième composition comprenant un ou plusieurs agents oxydants ;
* le ou les silicates choisis parmi les argiles de la famille des smectites, vermiculites, stévensites, chlorites se trouvant dans l'une et/ou l'autre des deux compositions, et de préférence dans la première ;
* le ou les agents alcalinisants optionnels se trouvant de préférence dans la première composition ;
* les compositions des deux compartiments étant destinées à être mélangées pour donner la composition selon l'invention, juste avant l'application sur les fibres kératiniques humaines, de telle sorte la composition résultant du mélange soit telle que définie précédemment avec notamment une concentration finale en silicates qui aille de 0,1 à 15% en poids.

L'invention concerne également un dispositif à deux compartiments comprenant dans l'un, une première composition comprenant un ou plusieurs corps gras, un ou plusieurs colorants d'oxydation, dans l'autre, une deuxième composition comprenant un ou plusieurs agents oxydants ; la première composition et/ou la deuxième composition, et de préférence la première composition, comprenant un ou plusieurs silicates particuliers et éventuellement un ou plusieurs agents alcalinisants, ce ou ces derniers étant de préférence dans la première composition ; les compositions des deux compartiments étant destinées à être mélangées pour donner la composition selon l'invention, juste avant l'application sur les fibres kératiniques humaines, de telle sorte la composition résultant du mélange soit telle que définie précédemment avec notamment une concentration finale en silicates qui aille de 0,1 à 15 % en poids.

L'invention concerne de plus un dispositif à trois compartiments renfermant :
* dans l'un, une première composition comprenant un ou plusieurs corps gras ;
* dans un autre, une deuxième composition renfermant un ou plusieurs colorants choisis parmi les colorants d'oxydation;
* et dans le dernier, une troisième composition comprenant un ou plusieurs agents oxydants ;
* la première composition et/ou la deuxième composition, et de préférence la première composition, comprenant un ou plusieurs silicates choisis parmi les argiles de la famille des smectites, vermiculites, stévensites, chlorites
* les compositions des trois compartiments étant destinées à être mélangées pour donner la composition selon l'invention, juste avant l'application sur les fibres kératiniques humaines, de telle sorte la composition résultant du mélange soit telle que définie précédemment avec notamment une concentration finale en silicates qui varie de 0,1 à 15% en poids.

Elle concerne également un dispositif à trois compartiments comprenant dans l'un, une première composition comprenant un ou plusieurs corps gras, dans un autre, une deuxième composition comprenant ou plusieurs colorants d'oxydation, et dans le dernier, une troisième composition comprenant un ou plusieurs agents oxydants ; la première, deuxième et/ou la troisième compositions comprenant un ou plusieurs silicates et éventuellement un ou plusieurs agents alcalinisants, ce ou ces derniers étant de préférence dans la seconde composition ; les compositions des trois compartiments étant destinées à être mélangées pour donner la composition selon l'invention, juste avant l'application sur les fibres kératiniques humaines de telle sorte la composition résultant du mélange soit telle que définie précédemment avec notamment une concentration finale en silicates qui varie de 0,1 à 15 % en poids.

Les exemples suivants servent à illustrer l'invention sans toutefois présenter un caractère limitatif.

### EXEMPLE1

On prépare les compositions suivantes (les quantités sont exprimées en g% de matières actives)

**Composition 1**

| | |
|---|---|
| Disteardimonium hectorite (Bentone 38 VCG) | 3 |
| Octyldodécanol | 11,5 |
| Distéarate de glycol | 8 |
| Huile de vaseline | 64,5 |
| Carbonate de propylène | 1 |
| Laureth-2 | 1 |
| Polysorbate 21 | 11 |

**Composition 2**

| | |
|---|---|
| Pentasodium pentetate | 1 |
| Métabisulfite de sodium | 0,7 |
| Monoéthanolamine | 14,5 |
| Toluène-2,5-diamine | 2,25 |
| Chlorhydrate de 2,4-diaminophénoxyéthanol | 0,05 |
| Résorcinol | 2 |
| m-aminophénol | 0,36 |
| Hydroxyéthylcellulose (Natrosol 250 HHR, Aqualon) | 1,5 |
| Hexylène glycol | 3 |
| Dipropylène glycol | 3 |
| Ethanol | 8,25 |
| Propylèneglycol | 6,2 |
| Acide ascorbique | 0,25 |
| eau | Qsp 100 |

**Composition 3**

| | |
|---|---|
| Pentasodium pentetate | 0,15 |
| Peroxyde d'hydrogène (solution aqueuse à 50 %) | 12 |
| Stannate de sodium | 0,04 |
| Acide phosphorique | Qs pH 2.2 |
| Pyrophosphate tétrasodique | 0,03 |
| Huile de vaseline | 20 |
| Polycondensat tétraméthyl hexaméthylènediamine / dichloro 1,3-propylène (solution aqueuse à 40% ; Hexadimethrine chloride) | 0,1 |
| Chlorure de polydiméthyl diallyl ammonium (solution aqueuse à 40 % non stabilisé, Polyquaternium-6 ) | 0,2 |
| Glycérine | 0,5 |
| Alcool cétylstéarylique (C16/C18 30/70 - NAFOL 1618F) | 8 |
| Alcool cétylstéarylique oxyéthyléné (33 OE) | 3 |
| Amide d'acides de colza oxyethylene (4 OE) | 1,2 |
| Vitamine E : DL-α-tocophérol | 0,1 |
| eau | Qsp 100 |

### Mode d'application

Les trois compositions détaillées ci-dessus sont mélangées au moment de l'emploi dans les proportions suivantes :
* 10 g de la composition 1
* 4 g de la composition 2
* 16 g de la composition 3.

Le mélange résultant est ensuite appliqué sur des mèches de cheveux naturels à 90 % de blancs, à raison de 10 g de mélange pour 1 g de cheveux.

Le mélange est laissé à température ambiante pendant 30 minutes.

Les cheveux sont ensuite rincés, lavés avec un shampooing standard et séchés.

On obtient des mèches châtain clair (évaluation visuelle).

### EXEMPLE 2 COMPARATIF

On prépare les compositions suivantes (les quantités sont exprimées en g% de matières actives) :

**Compositions A1 et A2 :**

| | A1 | A2 (invention) |
|---|---|---|
| Métasilicate de sodium | 3 | - |
| Disteardimonium hectorite | - | 3 |
| Carbonate de propylène | 1 | 1 |
| Octyldodécanol | 11,5 | 11,5 |
| Huile de vaseline | 64,5 | 64,5 |
| Distéarate d'éthylène glycol | 8 | 8 |
| Laureth-2 | 1 | 1 |
| Monolaurate de sorbitane oxyéthyléné (4 OE) | 11 | 11 |

**Composition B (en g) :**

| | |
|---|---|
| p-aminophénol | 1,5805 |
| Amino-3 chloro-2 méthyl-6 phénol | 2,2852 |
| Hydroxyéthyl cellulose (Natrosol 250 HHR) | 1,5 |
| Dipropylène glycol | 3 |
| Hexylène glycol | 3 |
| Propylène glycol | 6,2 |
| Monoéthanolamine | 15,04 |
| Alcool éthylique | 8,25 |
| Réducteurs, séquestrant | qs |
| Eau | Qs 100 |

**Composition C (en g) :**

| | |
|---|---|
| Peroxyde d'hydrogène | 6 |
| Alcool cétéarylique | 2,28 |
| Ceteareth-25 | 0,57 |
| Trideceth-2 carboxamide MEA | 0,85 |
| Glycérine | 0,5 |
| Stabilisants, séquestrants | Qs |
| Acide phosphorique | Qs pH = 2 |
| Eau | Qs 100 |

Au moment de l'emploi, on mélange 10g de composition A1 ou A2 avec 4 g de composition B et 15 g de composition C. Dans les deux cas, le pH de mélange est égal à 9,9 ± 0,1.

La viscosité de chacun des mélanges a été mesurée à l'aide d'un rhéomètre Rheomat RM180, à une température de 25°C (vitesse de rotation de 200 tours/mn, mobile 3).

| | A1 + B + C | A2 + B + C (invention) |
|---|---|---|
| Viscosité (mPa.s) | 850 | 1660 |

La viscosité du mélange obtenu avec la composition A2 est supérieure à celle du mélange obtenu avec la composition A1, ce qui signifie que le produit reste localisé où il a été appliqué, sans coulures.

Chaque mélange est ensuite appliqué sur des mèches de cheveux moyennement sensibilisés (Solubilité alcaline = 27), à raison de 14,5g de mélange pour 1 g de cheveux.

Après 35 minutes de pause à température ambiante, les cheveux sont rincés, lavés avec un shampooing standard et séchés.

Les mesures colorimétriques sont réalisées à l'aide d'un spectrocolorimètre Datacolor SF600X Spectraflash (illuminant D65, angle 10°, composantes spéculaires incluses).

### Chromaticité

| Mélange | a* | b* | C* |
|---|---|---|---|
| A1 + B + C | 23,33 | 21,88 | 31,99 |
| A2 + B + C (invention) | 23,36 | 25,30 | 34,43 |

On constate que le mélange obtenu avec la composition A2 selon l'invention conduit à une coloration significativement plus chromatique que le mélange obtenu avec la composition A1 hors invention.

## Revendications

1. Composition de coloration de fibres kératiniques humaines, **caractérisée en ce qu'**elle comprend, dans un milieu cosmétiquement acceptable:
(a) au moins 25% en poids d'un ou plusieurs corps gras ;
(b) 0,1 à 15% en poids d'un ou plusieurs silicates choisi parmi les argiles de la famille des smectites, vermiculites, stévensites, chlorites ;
(c) un ou plusieurs colorants choisis parmi les colorants d'oxydation ;
(d) un ou plusieurs agents oxydants.

2. Composition selon la revendication précédente, **caractérisée en ce que** le ou les corps gras sont choisis parmi les alcanes inférieurs en C₆-C₁₆, les huiles non siliconées d'origine animale, végétale, minérale ou synthétique, les alcools gras, les acides gras, les esters d'acide gras et/ou d'alcool gras, les cires non siliconées, les silicones.

3. Composition selon l'une quelconque des revendications 1 ou 2, **caractérisée en ce que** le ou les corps gras sont choisis parmi les composés liquides ou pâteux, et de préférence liquides à température ambiante et à pression atmosphérique.

4. Composition selon l'une quelconque des revendications précédentes dans laquelle le corps gras est choisi parmi les alcanes inférieurs en C₆-C₁₆, les huiles non siliconées d'origine végétale, minérale ou synthétique, les alcools gras, les acides gras, les esters d'acide gras et/ou d'alcool gras, ou leurs mélanges, et de préférence les alcanes inférieurs en C₆-C₁₆, les huiles non siliconées d'origine végétale, minérale ou synthétique, les alcools gras, les esters d'acide gras et/ou d'alcool gras, ou leurs mélanges.

5. Composition selon l'une quelconque des revendications précédentes, **caractérisée en ce que** la teneur en corps gras va de 25 à 80% en poids, de préférence de 25 à 65% en poids, plus particulièrement de 30 à 55% en poids par rapport au poids de la composition.

6. Composition selon l'une quelconque des revendications précédentes, **caractérisée en ce que** le ou les silicates sont choisis parmi les montmorillonites, les hectorites, les bentonites, les beidellites, les saponites.

7. Composition selon l'une quelconque des revendications précédentes, **caractérisée en ce que** les silicates sont modifiés avec un composé choisi parmi les amines quaternaires, les amines tertiaires, les acétates aminés, les imidazolines, les savons aminés, les sulfates gras, les alkyl aryl sulfonates, les oxides aminés, et leurs mélanges.

8. Composition selon l'une quelconque des revendications précédentes, **caractérisée en ce que** les silicates sont choisis parmi les quaternium-18 bentonites, les stéaralkonium bentonites, les quaternium-18/benzalkonium bentonites , les Quaternium-18 Hectorites.

9. Composition selon l'une quelconque des revendications précédentes, **caractérisée en ce qu'**elle comprend de 0,5 à 10% en poids de silicate(s), par rapport au poids de la composition.

10. Composition selon l'une quelconque des revendications précédentes, **caractérisée en ce qu'**elle comprend à titre de colorants d'oxydation, une ou plusieurs bases d'oxydation choisies parmi les paraphénylènediamines, les bis-phénylalkylènediamines, les para-aminophénols, les ortho-aminophénols, les bases hétérocycliques et leurs sels d'addition.

11. Composition selon la revendication précédente, **caractérisée en ce qu'**elle comprend un ou plusieurs coupleurs choisis parmi les méta-phénylènediamines, les méta-aminophénols, les méta-diphénols, les coupleurs naphtaléniques, les coupleurs hétérocycliques ainsi que leurs sels d'addition.

12. Composition selon l'une quelconque des revendications précédentes, **caractérisée en ce qu'**elle comprend un ou plusieurs agents alcalinisants choisis parmi l'ammoniaque, les carbonates ou bicarbonates alcalins, les hydroxydes de sodium ou de potassium et les amines organiques dont le pKb à 25°C est inférieur à 12, et de préférence inférieur à 10, encore plus avantageusement inférieur à 6.

13. Composition selon la revendication précédente, **caractérisée en ce que** l'agent alcalinisant est une amine organique choisie parmi les alcanolamines, de préférence la monoéthanolamine.

14. Composition selon la revendication 12, **caractérisée en ce que** l'agent alcalinisant est une amine organique choisie parmi les acides aminés basiques.

15. Procédé de coloration de fibres kératiniques humaines, **caractérisé en ce que** l'on applique la composition selon l'une quelconque des revendications précédentes.

16. Dispositif à deux compartiments renfermant dans l'un, une première composition comprenant un ou plusieurs corps gras, un ou plusieurs colorants choisis parmi les colorants d'oxydation ; dans l'autre, une deuxième composition comprenant un ou plusieurs agents oxydants ; la première composition et/ou la deuxième composition, et de préférence la première composition, comprenant un ou plusieurs silicates choisis parmi les argiles de la famille des smectites, vermiculites, stévensites, chlorites; le ou les agents alcalinisants optionnels se trouvant de préférence dans la première composition ; les compositions des deux compartiments étant destinées à être mélangées pour donner la composition selon l'invention, juste avant l'application sur les fibres kératiniques humaines, de telle sorte que la composition résultant du mélange soit telle que définie dans l'une quelconque des revendications 1 à 14.

17. Dispositif à trois compartiments renfermant dans l'un, une première composition comprenant un ou plusieurs corps gras ; dans un autre, une deuxième composition comprenant un ou plusieurs colorants choisis parmi les colorants d'oxydation; et dans le dernier, une troisième composition comprenant un ou plusieurs agents oxydants ; la première, deuxième et/ou la troisième compositions comprenant un ou plusieurs silicates choisis parmi les argiles de la famille des smectites, vermiculites, stévensites, chlorites ; les compositions des trois compartiments étant destinées à être mélangées pour donner la composition selon l'invention, juste avant l'application sur les fibres kératiniques humaines de telle sorte que la composition résultant du mélange soit telle que définie dans l'une quelconque des revendications 1 à 14.

## Patentansprüche

1. Zusammensetzung zum Färben von menschlichen Keratinfasern, **dadurch gekennzeichnet, dass** sie in einem kosmetisch unbedenklichen Medium Folgendes umfasst:
(a) mindestens 25 Gew.-% einer oder mehrerer Fettsubstanzen;
(b) 0,1 bis 15 Gew.-% eines oder mehrerer Silikate, die aus Tonen der Familie der Smektite, Vermiculite, Stevensite und Chlorite ausgewählt sind;
(c) einen oder mehrere Farbstoffe, die aus Oxidationsfarbstoff und ausgewählt sind;
(d) ein oder mehrere Oxidationsmittel.

2. Zusammensetzung nach dem vorhergehenden Anspruch, **dadurch gekennzeichnet, dass** die Fettsubstanz bzw. die Fettsubstanzen aus C₆-C₁₆-Niederalkanen, Nichtsilikonölen tierischer, pflanzlicher, mineralischer oder synthetischer Herkunft, Fettalkoholen, Fettsäuren, Fettsäureestern und/oder Fettalkoholestern, Nichtsilikonwachsen und Silikonen ausgewählt ist bzw. sind.

3. Zusammensetzung nach Anspruch 1 oder 2, **dadurch gekennzeichnet, dass** die Fettsubstanz bzw. die Fettsubstanzen aus Verbindungen, die bei Umgebungstemperatur und Normaldruck flüssig oder pastös und vorzugsweise flüssig sind, ausgewählt ist bzw. sind.

4. Zusammensetzung nach einem der vorhergehenden Ansprüche, wobei die Fettsubstanz aus C₆-C₁₆-Niederalkanen, Nichtsilikonölen pflanzlicher, mineralischer oder synthetischer Herkunft, Fettalkoholen, Fettsäuren, Fettsäureestern und/oder Fettalkoholestern oder Mischungen davon und vorzugsweise C₆-C₁₆-Niederalkanen, Nichtsilikonölen pflanzlicher, mineralischer oder synthetischer Herkunft, Fettalkoholen, Fettsäureestern und/oder Fettalkoholestern oder Mischungen davon ausgewählt ist.

5. Zusammensetzung nach einem der vorhergehenden Ansprüche, **dadurch gekennzeichnet, dass** der Gehalt an Fettsubstanz 25 bis 80 Gew.-%, vorzugsweise 25 bis 65 Gew.-%, spezieller 30 bis 55 Gew.-%, bezogen auf das Gewicht der Zusammensetzung, beträgt.

6. Zusammensetzung nach einem der vorhergehenden Ansprüche, **dadurch gekennzeichnet, dass** das Silikat bzw. die Silikate aus Montmorilloniten, Hectoriten, Bentoniten, Beidelliten und Saponiten ausgewählt ist bzw. sind.

7. Zusammensetzung nach einem der vorhergehenden Ansprüche, **dadurch gekennzeichnet, dass** die Silikate mit einer Verbindung, die aus quaternären Aminen, tertiären Aminen, Aminacetaten, Imidazolinen, Aminseifen, Fettsulfaten, Alkylarylsulfonaten, Aminoxiden und Mischungen davon ausgewählt ist, modifiziert sind.

8. Zusammensetzung nach einem der vorhergehenden Ansprüche, **dadurch gekennzeichnet, dass** die Silikate aus Quaternium-18-bentoniten, Stearalkoniumbentoniten, Quaternium-18-/Benzalkoniumbentoniten und Quaternium-18-hectoriten ausgewählt sind.

9. Zusammensetzung nach einem der vorhergehenden Ansprüche, **dadurch gekennzeichnet, dass** sie 0,5 bis 10 Gew.-%Silikat(e), bezogen auf das Gewicht der Zusammensetzung, umfasst.

10. Zusammensetzung nach einem der vorhergehenden Ansprüche, **dadurch gekennzeichnet, dass** sie als Oxidationsfarbstoffe eine oder mehrere Oxidationsbasen, die aus para-Phenylendiaminen, Bisphenylalkylendiaminen, para-Aminophenolen, ortho-Aminophenolen, heterocyclischen Basen und Additionssalzen davon ausgewählt sind, umfasst.

11. Zusammensetzung nach dem vorhergehenden Anspruch, **dadurch gekennzeichnet, dass** sie einen oder mehrere Kuppler, die aus meta-Phenylendiaminen, meta-Aminophenolen, meta-Diphenolen, naphthalin-Kupplern, heterocyclischen Kupplern sowie Additionssalzen davon ausgewählt sind, umfasst.

12. Zusammensetzung nach einem der vorhergehenden Ansprüche, **dadurch gekennzeichnet, dass** sie ein oder mehrere Alkalisierungsmittel, die aus Ammoniak, Alkalicarbonaten oder -hydrogencarbonaten, Natrium- oder Kaliumhydroxid und organischen Aminen, deren pKb-Wert bei 25 °C unter 12 und vorzugsweise unter 10 und noch vorteilhafter unter 6 liegt, umfasst.

13. Zusammensetzung nach dem vorhergehenden Anspruch, **dadurch gekennzeichnet, dass** es sich bei dem Alkalisierungsmittel um ein organisches Amin, das aus Alkanolaminen ausgewählt ist, vorzugsweise Monoethanolamin, handelt.

14. Zusammensetzung nach Anspruch 12, **dadurch gekennzeichnet, dass** es sich bei dem Alkalisierungsmittel um ein organisches Amin, das aus basischen Aminosäuren ausgewählt ist, handelt.

15. Verfahren zum Färben von menschlichen Keratinfasern, **dadurch gekennzeichnet, dass** man die Zusammensetzung nach einem der vorhergehenden Ansprüche aufbringt.

16. Vorrichtung mit zwei Kompartimenten, die in einem Kompartiment eine erste Zusammensetzung, die eine oder mehrere Fettsubstanzen und einen oder mehrere Farbstoffe, die aus oxidationsfarbstoffen ausgewählt sind; und in dem anderen Kompartiment eine zweite Zusammensetzung, die ein oder mehrere Oxidationsmittel umfasst; enthält; wobei die erste Zusammensetzung und/oder die zweite Zusammensetzung und vorzugsweise die erste Zusammensetzung ein oder mehrere Silikate, die aus Tonen der Familie der Smektite, Vermiculite, Stevensite und Chlorite ausgewählt sind, umfasst bzw. umfassen; wobei das fakultative Alkalisierungsmittel bzw. die fakultativen Alkalisierungsmittel vorzugsweise in der ersten Zusammensetzung vorliegt bzw. vorliegen; wobei die Zusammensetzungen in den beiden Kompartimenten zum Mischen zu der erfindungsgemäßen Zusammensetzung unmittelbar vor dem Aufbringen auf die menschlichen Keratinfasern vorgesehen sind, so dass die sich aus dem Mischen ergebende Zusammensetzung wie in einem der Ansprüche 1 bis 14 definiert ist.

17. Vorrichtung mit drei Kompartimenten, die in einem Kompartiment eine erste Zusammensetzung, die eine oder mehrere Fettsubstanzen umfasst; in einem anderen Kompartiment eine zweite Zusammensetzung, die einen oder mehrere Farbstoffe, die aus Oxidationsfarbstoffen ausgewählt sind; und in dem letzten Kompartiment eine dritte Zusammensetzung, die einen oder mehrere Oxidationsmittel umfasst; enthält; wobei die erste, zweite und/oder dritte Zusammensetzung ein oder mehrere Silikate, die aus Tonen der Familie der Smektite, Vermiculite, Stevensite und Chlorite ausgewählt sind, umfasst bzw. umfassen; wobei die Zusammensetzungen in den drei Kompartimenten zum Mischen zu der erfindungsgemäßen Zusammensetzung unmittelbar vor dem Aufbringen auf menschliche Keratinfasern vorgesehen sind, so dass die sich aus dem Mischen ergebende Zusammensetzung wie in einem der Ansprüche 1 bis 14 definiert ist.

## Claims

1. Composition for dyeing human keratin fibres, **characterized in that** it comprises, in a cosmetically acceptable medium:
(a) at least 25% by weight of one or more fatty substances;
(b) 0.1% to 15% by weight of one or more silicates chosen from clays of the family of smectites, vermiculites, stevensites and chlorites;
(c) one or more dyes chosen from oxidation dyes;
(d) one or more oxidizing agents.

2. Composition according to the preceding claim, **characterized in that** the fatty substances are chosen from C₆-C₁₆ lower alkanes, non-silicone oils of animal, plant, mineral or synthetic origin, fatty alcohols, fatty acids, esters of a fatty acid and/or of a fatty alcohol, non-silicone waxes and silicones.

3. Composition according to either of Claims 1 and 2, **characterized in that** the fatty substance(s) is (are) chosen from compounds that are liquid or pasty and preferably liquid at room temperature and at atmospheric pressure.

4. Composition according to any one of the preceding claims, in which the fatty substance is chosen from C₆-C₁₆ lower alkanes, non-silicone oils of plant, mineral or synthetic origin, fatty alcohols, fatty acids, and esters of a fatty acid and/or of a fatty alcohol, or mixtures thereof, and preferably C₆-C₁₆ lower alkanes, non-silicone oils of plant, mineral or synthetic origin, fatty alcohols, and esters of a fatty acid and/or of a fatty alcohol, or mixtures thereof.

5. Composition according to any one of the preceding claims, **characterized in that** the content of fatty substances ranges from 25% to 80% by weight, preferably from 25% to 65% by weight and even more particularly from 30% to 55% by weight relative to the weight of the composition.

6. Composition according to any one of the preceding claims, **characterized in that** the silicate(s) is (are) chosen from montmorillonites, hectorites, bentonites, beidellites and saponites.

7. Composition according to any one of the preceding claims, **characterized in that** the silicates are modified with a compound chosen from quaternary amines, tertiary amines, amine acetates, imidazolines, amine soaps, fatty sulfates, alkyl aryl sulfonates and amine oxides, and mixtures thereof.

8. Composition according to any one of the preceding claims, **characterized in that** the silicates are chosen from quaternium-18 bentonites, stearalkonium bentonites, quaternium-18/benzalkonium bentonites and quaternium-18 hectorites.

9. Composition according to any one of the preceding claims, **characterized in that** it comprises from 0.5% to 10% by weight of silicate(s), relative to the weight of the composition.

10. Composition according to any one of the preceding claims, **characterized in that** it comprises, as oxidation dyes, one or more oxidation bases chosen from para-phenylenediamines, bis(phenyl)alkylenediamines, para-aminophenols, ortho-aminophenols and heterocyclic bases, and the addition salts thereof.

11. Composition according to the preceding claim, **characterized in that** it comprises one or more couplers chosen from meta-phenylenediamines, meta-aminophenols, meta-diphenols, naphthalene-based couplers and heterocyclic couplers, and also the addition salts thereof.

12. Composition according to any one of the preceding claims, **characterized in that** it comprises one or more basifying agents chosen from aqueous ammonia, alkali metal carbonates or bicarbonates, sodium hydroxide, potassium hydroxide and organic amines whose pKb at 25°C is less than 12, preferably less than 10 and even more advantageously less than 6.

13. Composition according to the preceding claim, **characterized in that** the basifying agent is an organic amine chosen from alkanolamines, preferably monoethanolamine.

14. Composition according to Claim 12, **characterized in that** the basifying agent is an organic amine chosen from basic amino acids.

15. Process for dyeing human keratin fibres, **characterized in that** the composition according to any one of the preceding claims is applied.

16. Two-compartment device including, in one, a first composition comprising one or more fatty substances, optionally one or more dyes chosen from oxidation dyes; in the other, a second composition comprising one or more oxidizing agents; the first composition and/or the second composition and preferably the first composition, comprising one or more silicates chosen from clays of the family of smectites, vermiculites, stevensites and chlorites; the optional basifying agent(s) preferably being in the first composition; the compositions of the two compartments being intended to be mixed together to give the composition according to the invention just before application to the human keratin fibres, such that the composition resulting from the mixing is as defined in any one of Claims 1 to 14.

17. Three-compartment device including, in one, a first composition comprising one or more fatty substances, in another, a second composition comprising one or more dyes chosen from oxidation dyes; and in the final compartment, a third composition comprising one or more oxidizing agents; the first, second and/or third composition comprising one or more silicates chosen from clays of the family of smectites, vermiculites, stevensites and chlorites; the compositions of the three compartments being intended to be mixed together to give the composition according to the invention, just before application to the human keratin fibres, such that the composition resulting from the mixing is as defined in any one of Claims 1 to 14.
